# Europäisches Patentamt

# European Patent Office

# Office européen des brevets

(19)

(11) Numéro de publication: **0 043 319**
**B1**

(12) **FASCICULE DE BREVET EUROPEEN**

(45) Date de publication du fascicule du brevet:
25.04.84

(51) Int. Cl.³: **A 61 K 9/52**, A 61 K 9/22

(21) Numéro de dépôt: 81401023.7

(22) Date de dépôt: 25.06.81

(54) Support inerte en copolymère réticulé, son procédé de préparation et son utilisation pour la réalisation de médicaments retard.

(30) Priorité: 30.06.80 FR 8014529

(43) Date de publication de la demande:
06.01.82 Bulletin 82/1

(45) Mention de la délivrance du brevet:
25.04.84 Bulletin 84/17

(84) Etats contractants désignés:
AT BE CH DE FR GB IT LI LU NL SE

(73) Titulaire: **COMMISSARIAT A L'ENERGIE ATOMIQUE Etablissement de Caractère Scientifique Technique et Industriel, B.P. 510, F-75752 Paris Cedex 15 (FR)**

(72) Inventeur: **Berthet, Jeanne, 1, Allée du Roussillon, F-78140 Velizy Villacoublay (FR)**
Inventeur: **Blin, Marie-Françoise, 86, Avenue de Paris, F-78000 Versailles (FR)**
Inventeur: **Gaussens, Gilbert, 11, rue Jean Brunet, F-92190 Meudon (FR)**

(74) Mandataire: **Mongrédien, André et al, c/o BREVATOME 25, rue de Ponthieu, F-75008 Paris (FR)**

(56) Documents cités:
FR - A - 2 250 793
FR - A - 2 323 756
FR - A - 2 433 949
GB - A - 1 426 101
US - A - 3 857 932
US - A - 3 901 967

CHEMICAL ABSTRACTS, vol. 91, no. 4, 23 juillet 1979 page 419, abrégé 27254b Columbus, Ohio, USA H. KALA et al. "Preparation and testing of drug-loaded polymerbeads"
MERCK INDEX, Encyclopedia of Chemicals & Drugs 9e edition, Rayway, Merck & Co, 1976, USA 1980
Acta Pharm. Technol. Suppl. 1979 (7), pages 177-184

Le dossier contient des informations techniques présentées postérieurement au dépôt de la demande et ne figurant pas dans le présent fascicule.

Support inerte en copolymère réticulé, son procédé de préparation et son utilisation
pour la réalisation de médicaments retard

La présente invention a pour objet un support inerte en copolymère réticulé, capable d'absorber puis de libérer progressivement une substance pharmaceutiquement active, son procédé de préparation et son utilisation pour la réalisation de médicaments-retard.

Jusqu'à présent, pour la réalisation de médicaments-retard, c'est-à-dire de médicaments capables de libérer progressivement une substance pharmaceutiquement active, on a utilisé des procédés consistant à enrober, encapsuler ou microencapsuler la substance pharmaceutiquement active dans un matériau capable de ralentir sa diffusion dans le milieu extérieur.

Cependant, ces procédés présentent l'inconvénient d'être d'une mise en œuvre difficile et de ne pas permettre d'assurer un contrôle satisfaisant des cinétiques de désorption de la substance pharmaceutiquement active ainsi enrobée ou encapsulée.

On connaît également par le brevet français 2 250 793 du 9 novembre 1973 un procédé de réalisation de produits capables de libérer une substance chimique, qui est basé sur l'emploi de supports en polymère hydrophobe comportant des inclusions hydrophiles polymérisées et greffées sur le polymère hydrophobe. Dans ce cas, on emmagasine la substance dans le support par absorption à partir d'une solution, ce qui constitue un avantage par rapport aux procédés d'enrobage ou d'encapsulation, car l'étape d'emmagasinage est d'une réalisation plus aisée. Par ailleurs, l'emploi de ces supports permet de mieux contrôler la cinétique de désorption de la substance retenue par le support.

Le brevet français FR-A-2 323 756 illustre des supports en polymère utilisables pour emprisonner des agents tensio-actifs, ces supports sont à base de polymère ou de copolymère acrylique réticulé, mais dans ce cas, on introduit l'agent tensio-actif au cours de la fabrication du polymère ou du copolymère, ce qui ne convient pas pour la réalisation de médicaments-retard, car il est préférable dans ce cas, d'introduire le médicament après la fabrication du support, afin de ne pas modifier les caractéristiques de celui-ci.

Le brevet américain US-A-3 857 932 décrit lui aussi des polymères acryliques ou méthacryliques pour médicaments à libération progressive qui sont préparés à partir de monomères hydrophiles. De tels supports présentent des caractéristiques satisfaisantes d'absorption de la substance pharmaceutique, mais ils ne présentent pas une bonne cinétique de désorption, car la totalité du médicament est pratiquement libérée en une heure.

Le brevet anglais GB-A-1 426 101 illustre également l'utilisation d'homopolymères et de copolymères acryliques hydrophiles pour retenir un microorganisme dans une matrice. Comme on l'a vu précédemment, de tels copolymères ne conviennent pas pour la réalisation de médicaments-retard, en raison de leur cinétique de désorption.

L'article H. KALA et al. Acta Pharmaceutica Technologica supplément 1979, 7, pages 177–184, illustre l'emploi de copolymères à base d'acide acrylique pour fixer des produits pharmaceutiques par liaison chimique entre la fonction acide de l'acide acrylique et le produit actif. Cependant, bien que le taux de fixation du produit actif soit satisfaisant, on ne peut obtenir dans ce cas, une désorption satisfaisante du produit stocké.

La présente invention a pour objet des supports inertes en copolymère réticulé, capables de retenir puis de libérer progressivement une substance pharmaceutiquement active, qui présentent en raison de leur composition particulière des propriétés améliorées par rapport aux supports polymères précités, notamment en ce qui concerne leur capacité d'absorption de la substance et leur possibilité d'assurer un contrôle des cinétiques de désorption de la substance retenue.

L'invention a également pour objet des supports en copolymère réticulé qui sont plus particulièrement adaptés à la rétention de substances pharmaceutiquement actives telles que la codéine et le kétoprofène, c'est-à-dire l'acide 2-(3-benzoylphényl)-propionique.

Selon l'invention, le support inerte en copolymère réticulé capable d'absorber puis de libérer progressivement une substance pharmaceutiquement active, se caractérise en ce qu'il est constitué par une poudre ayant une granulométrie de 20 à 700 µm, d'un copolymère réticulé comprenant:

— de 30 à 80% en poids d'acrylate et/ou de méthacrylate d'alkyle monoinsaturé;
— de 5 à 68% en poids d'acide acrylique et/ou méthacrylique, et
— de 2 à 15% en poids d'ester acrylique et/ou méthacrylique choisi dans le groupe comprenant le diacrylate de diéthylène glycol, le diacrylate de tétraéthylène glycol (DIATEG), le diacrylate de polyéthylèr glycol (DIAPEG), le triacrylate de triméthylol propane (TATMP) et le triméthacrylate de triméthylol propane (TMATMP).

Selon l'invention, dans les acrylates ou méthacrylates d'alkyle utilisés, les radicaux alkyle ont généralement de 1 à 12 atomes de carbone.

Avantageusement, les acrylates ou méthacrylates d'alkyle monoinsaturés sont choisis dans le groupe comprenant les acrylates de butyle (ABu), d'hexyle et d'heptyle, et les méthacrylates de méthyle (MAM), d'éthyle de butyle (MABu), d'hexyle et d'heptyle (MAHept).

Selon l'invention, on entend par ester acrylique ou méthacrylique bi- ou trifonctionnel un ester comportant deux ou trois doubles liaisons.

De préférence, le copolymère réticulé comprend:

— de 30 à 80% de méthacrylate de butyle ou d'heptyle,
— de 5 à 68% d'acide acrylique,
— de 2 à 15% de triméthacrylate ou triacrylate de triméthylolpropane, ou de diacrylate de tétraéthylèneglycol.

Selon l'invention, lorsque le support est destiné à la réalisation de médicaments-retard administrables par voie orale ou rectale, la poudre est sous la forme de particules ayant une granulométrie telle que lesdites particules ne soient pas retenues dans les replis des parois intestinales.

Du point de vue pratique, la taille moyenne de ces particules est inférieure à 700 μm, supérieure à 20 μm, et située plus particulièrement à l'intérieur d'une gamme de 100 à 400 μm lorsque le support est destiné à la réalisation de médicaments administrés par voie orale, et de 20 à 50 μm lorsque le support est destiné à la réalisation de médicaments administrés par voie rectale.

L'invention a également pour objet un procédé de préparation d'un tel support inerte en copolymère réticulé.

Ce procédé se caractérise en ce que l'on polymérise un mélange de monomères acryliques et/ou méthacryliques comprenant un acrylate et/ou un méthacrylate d'alkyle monoinsaturé, de l'acide acrylique et/ou de l'acide méthacrylique, et un ester acrylique et/ou méthacrylique choisi dans le groupe précité, suivant un mécanisme de polymérisation radicalaire.

De préférence, on réalise la polymérisation en émulsion sous rayonnement ionisant.

Avantageusement, on soumet le copolymère réticulé ainsi obtenu à une irradiation complémentaire au moyen de rayonnements ionisants pour adapter son taux de réticulation.

La dose d'irradiation intégrée utilisée pour réaliser la polymérisation et la réticulation est généralement d'environ 0,9 à 8 Mrad, soit 9000 à 80 000 Gy.

Les supports selon l'invention peuvent être utilisés pour la réalisation de médicaments-retard.

Pour la réalisation de tels médicaments, on maintient une substance pharmaceutiquement active au contact du support en copolymère réticulé, sous la forme de particules, pendant une durée suffisante pour réaliser l'absorption de la quantité désirée de substance active sur et dans le support.

De préférence, on maintient les particules du support en suspension au sein d'une solution, de préférence aqueuse ou alcoolique, de la substance pharmaceutiquement active, contenant avantageusement 10 à 60 mg de substance pour 100 ml, pendant une durée suffisante pour assurer l'absorption de la quantité désirée de ladite substance, puis on élimine la solution et on sèche ou lyophilise les particules du support ayant retenu la substance pharmaceutiquement active jusqu'à l'obtention d'un poids constant, pour éliminer le solvant absorbé dans le support.

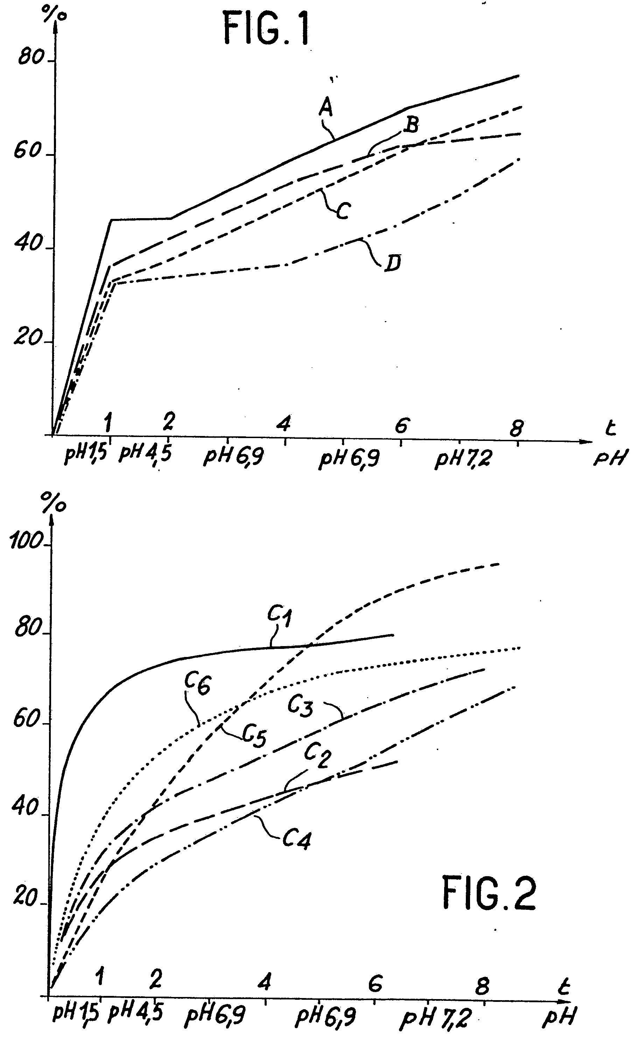

D'autres caractéristiques et avantages de l'invention apparaîtront mieux à la lecture de la description qui suit et des exemples donnés à titre illustratif et non limitatif, en référence au dessin annexé sur lequel les figures 1 à 7 sont des représentations graphiques illustrant la libération progressive de la substance pharmaceutiquement active en fonction du temps (en heures).

Pour préparer les supports inertes en matériau polymère capables d'absorber puis de libérer progressivement une substance pharmaceutiquement active, conformes à l'invention, on s'y prend comme suit ou de façon équivalente.

On prépare la structure polymère réticulée servant de support soit par polymérisation en masse, soit par polymérisation en émulsion, de préférence sous irradiation ionisante.

Dans le premier cas, on soumet une quantité suffisante de chacun des deux types de monomères susmentionnés, c'est-à-dire des monomères acryliques et/ou méthacryliques monoinsaturés et des monomères acryliques et/ou méthacryliques bi- ou trifonctionnels, sous un vide d'au moins 1,35 Pa ou sous atmosphère d'azote, à une irradiation ionisante, par exemple au moyen de rayons ultraviolets, de rayons X, de rayons $\alpha$, $\beta$, ou $\gamma$, ou encore de faisceaux d'électrons accélérés, notamment de rayonnement $\gamma$, réalisant ainsi la polymérisation et la réticulation grâce au monomère polyfonctionnel capable de créer un réseau tri-dimensionnel.

Dans le deuxième cas, on prépare une émulsion de la phase organique, constituée par le mélange de monomères, dans une phase aqueuse faiblement acide, de pH voisin de 3 à 4 qui contient en solution un sel, notamment le sulfate de sodium, à sa concentration maximum pour éviter le passage des monomères solubles dans l'eau vers cette phase aqueuse; la phase aqueuse contient par ailleurs un agent inhibiteur de la polymérisation desdits monomères, notamment le sel de Mohr, ainsi qu'un agent favorisant l'établissement de l'émulsion, notamment l'acide polyacrylique.

Le rapport pondéral de la phase aqueuse sur la phase organique est voisin de 1.

L'émulsion est obtenue très rapidement par mélange énergique des susdites phases aqueuse et organique à la température ordinaire.

Tout comme dans le premier cas, c'est à l'aide d'une irradiation ionisante à laquelle on soumet l'émulsion que l'on réalise la polymérisation et la réticulation. Ici encore, on utilise avantageusement le

3

rayonnement γ.

Dans les deux types de polymérisation, la quantité de monomère polyfonctionnel mis en œuvre représente avantageusement environ 2 à 15% en poids de la masse totale des monomères soumis à l'irradiation.

Le débit de dose appliqué est avantageusement d'environ 0,1 à 0,25 Mrad/heure. La dose intégrée nécessaire pour réaliser la polymérisation et la réticulation est d'environ 0,9 à 8 Mrad.

La réticulation s'effectue sous l'action du rayonnement ionisant et du monomère acrylique et/ou méthacrylique bi- ou trifonctionnel.

Le taux de réticulation dont dépendent la quantité de substance active retenue et la programmation ou allure de la libération progressive de celle-ci, est fonction de la valeur choisie pour la dose intégrée de rayonnements ionisants appliqués.

Il est difficile de donner une loi s'appliquant à tous les cas et, par conséquent, on déterminera cas par cas et par essais préalables pour des monomères et une substance active de départ donnés, le taux de réticulation, c'est-à-dire la dose intégrée devant être appliquée, pour retenir une quantité donnée de substance active par unité de masse de support.

A titre illustratif, on indique plus loin, pour un copolymère donné, un exemple de variation du taux illustré par la quantité de substance active retenue et par l'allure des courbes de la désorption, c'est-à-dire de la libération progressive de celle-ci pour différentes doses intégrées appliquées.

Une fois la polymérisation et la réticulation terminées, on broie, lorsque cela est nécessaire, le copolymère et on isole, par exemple par tamisage, la fraction ayant la granulométrie appropriée, par exemple la fraction ayant une granulométrie de 20 à 700 μm, et en particulier une granumométrie de 200 à 400 μm lorsque le support est destiné à la fabrication de médicaments administrés par voie orale, ou une granulométrie de 20 à 50 μm lorsque le support est destiné à la réalisation de médicaments administrés par voie rectale.

Ces supports en poudre peuvent être utilisés ensuite pour la préparation de médicaments-retard. Dans ce cas, on réalise l'absorption de la substance active dans la structure du support de la façon suivante.

On maintient une quantité de particules de copolymère déterminée avec précision, en suspension pendans 2 à 120 heures, dans une solution, de préférence à une température de 20 à 75°C, de la substance active dans l'eau ou dans un alcool, le rapport »poids de copolymère en g/volume de solution de substance active en ml« étant de 1/2 à 1/25 et la concentration en substance active de la solution de 10 à 60 g pour 100 ml.

Après cette opération, on filtre la suspension pour séparer les particules du support de la solution, puis on élimine le solvant en séchant ces particules à l'étuve à une température telle que la substance active ne soit pas dégradée, jusqu'à l'obtention d'un poids constant.

Pour illustrer l'efficacité de la forme galénique retard du médicament ainsi obtenu, on détermine la cinétique de désorption de la substance active qui peut être effectuée à pH variable simulant le transit digestif ou à pH fixe.

Des exemples de cinétique de désorption sont donnés plus loin.

De préférence, les taux de libération de la substance pharmaceutiquement active en fonction du temps et du pH sont dans le cas de la codéine:

— contact   1 heure  à ph 1,5
       30% < libération < 45%
— contact   1 heure  à pH 1,5
           +1 heure  à pH 4,5
           +2 heures à pH 6,9
       60% < libération < 70%
— contact   1 heure  à pH 1,5
           +1 heure  à pH 4,5
           +4 heures à pH 6,9
           +2 heures à pH 7,2
       libération > 70%

On a montré par ailleurs, comme également illustré plus loin, que les substances actives retenues dans les médicaments-retard ainsi obtenues plus désorbées, ne se trouvent pas altérées du point de vue chimique.

Ces médicaments-retard qui peuvent être administrés par les voies orale et rectale sont présentés avantageusement sous formes de gélules ou de suppositoires renfermant la quantité nécessaire de substance pharmaceutiquement active.

Les exemples suivants illustrent l'invention en rapport avec des modes de réalisation avantageux.

4

## Exemple 1

### a) Préparation du support polymère réticulé

Dans un réacteur en pyrex équipé d'un dispositif d'agitation, on soumet, sous atmosphère d'azote (balayage), à un débit de dose de 0,224 Mrad/heure à la température ambiante, une émulsion du mélange de monomères suivants (phase organique):

55 parties en poids de MABu;
35 parties en poids d'acide acrylique;
10 parties en poids de TMATMP

dans une phase aqueuse composée de 30% en poids de sulfate de sodium dans 70% en poids d'une solution d'acide sulfurique 0,01 N à laquelle on ajoute une quantité de sel de Mohr correspondant à 2% de la quantité d'acide acrylique présent dans la phase organique.

Le rapport pondéral de la phase aqueuse sur la phase organique est égal à 1.

L'émulsion est amorcée par l'addition de 0,5% en poids, par rapport au poids de la phase aqueuse, d'acide polyacrylique ajouté à l'émulsion sous forme de poudre de granulométrie de 200 à 400 μm. La polymérisation nécessite une dose intégrée de 0,9 Mrad.

On améliore la réticulation par des irradiations supplémentaires.

On a préparé les copolymères réticulés correspondant aux doses intégrées complémentaires suivantes:

1 Mrad
2 Mrad
4 Mrad

Après irradiation, on sèche les particules de copolymère obtenues et on isole la fraction de particules ayant une granulométrie de 250 à 400 μm.

### b) Absorption de la codéine

On maintient sous agitation pendant 5 heures une suspension de 1 gramme de particules de copolymère ainsi obtenues ayant une granulométrie de 250 à 400 μm dans une solution à 20 g/100 ml de codéine dans un mélange eau/éthanol en proportions de 9/1, à une température voisine de 60° C.

Les quantités de codéine absorbée sont les suivantes:

| | |
|---|---|
| Copolymère réticulé A (irradiation de 0,9 Mrad) en poids par rapport au produit final | 29% |
| Copolymère réticulé B (irradiation de 1,9 Mrad) en poids par rapport au produit final | 35,5% |
| Copolymère réticulé C (irradiation de 2,9 Mrad) en poids par rapport au produit final | 40% |
| Copolymère réticulé D (irradiation de 4,9 Mrad) en poids par rapport au produit final. | 41,5% |

### c) Désorption à pH variable

Pour cette expérience, un échantillon de 0,1 g de particules de chacun des copolymères A à D est maintenu en suspension par agitation de va-et-vient, pendant les durées indiquées ci-après, successivement dans quatre solutions, de pH respectivement croissant, à une température de 37 ± 2° C, à savoir:

— Première solution à pH 1,5 pendant la première heure;
— Deuxième solution à pH 4,5 pendant la deuxième heure;
— Troisième solution à pH 6,9 pendant les troisième, quatrième, cinquième et sixième heures;
— Quatrième solution à pH 7,2 pendant les septième et huitième heures.

Des prélèvements de solution sont effectués à chaque changement de pH et la concentration en substance active est déterminée par spectrophotométrie.

Les solutions des différents pH sont préparées, en ce qui concerne celle de pH 1,5, à partir de HCl et de NaOH 1N, et, en ce qui concerne celle de pH 4,5, puis celle de pH 6,9 et enfin celle de pH 7,2, à partir de phosphate monopotassique et de HCl 1N.

Le résultat de cette désorption est illustré par les courbes A à D (qui correspondent respectivement aux copolymères A à D) apparaissant à la figure 1 et traduisant la variation de la quantité de substance active (exprimée en % en poids par rapport à la quantité totale de substance active) désorbée en fonction du temps t (en heures) et du pH.

A l'examen de ces courbes, on constate que la vitesse de désorption décroît lorsque la dose d'irradiation augmente.

On donne six autres exemples dans lesquels la substance active est la codéine.

## Exemple 2

La composition du polymère est la suivante:

| | |
|---|---|
| méthacrylate de butyle: | 35% |
| acide acrylique: | 55% |
| triméthacrylate de triméthylolpropane: | 10% |

et sa granulométrie est de 250 à 400 μm.

Il a été obtenu par polymérisation en masse sous vide à un débit de dose de 0,1 Mrad/h$^{-1}$, la dose d'irradiation étant de 2,4 Mrad.

Les conditions expérimentales de la phase d'absorption sont les suivantes:

| | |
|---|---|
| quantité de polymère: | 1 gramme |
| volume de solution: | 8 ml |
| solvant: | éthanol |
| concentration de codéine: | 22 g pour 100 ml |
| durée: | dix-huit heures |
| température: | 50° C |

La quantité de codéine absorbée est de 54%.

La cinétique de désorption, à pH variable, est représentée par la courbe $C_1$ de la figure 2.

## Exemple 3

La composition du polymère est la suivante:

| | |
|---|---|
| méthacrylate de butyle: | 55% |
| acide acrylique: | 35% |
| triméthacrylate de triméthylolpropane: | 10% |

et sa granulométrie est de 250 à 400 μm.

Les conditions de la polymérisation sont celles de l'exemple 2.

Les conditions expérimentales de la phase d'absorption sont identiques à celles de l'exemple 2.

La cinétique de désorption, à pH variable, est représentée par la courbe $C_2$ montrée figure 2.

## Exemple 4

La composition du polymère est la suivante:

| | |
|---|---|
| méthacrylate de butyle: | 55% |
| acide acrylique: | 35% |
| triméthacrylate de triméthylolpropane: | 10% |

et sa granulométrie est de 250 à 400 μm.

Les conditions de la polymérisations sont celles de l'exemple 2.

Les conditions expérimentales de la phase d'absorption sont les suivantes:

| | |
|---|---|
| quantité de polymère: | 1 gramme |
| volume de solution: | 8 ml |
| solvant: | eau |

concentration de codéine:         12,5 g pour 100 ml
durée:         trois heures
température:         75° C

La quantité de codéine absorbée est de 45,5%.
La cinétique de désorption, à pH variable, est représentée par la courbe $C_3$ de la figure 2.

### Exemple 5

La composition du polymère est la suivante:

méthacrylate de butyle:         55%
acide acrylique:         35%
triméthacrylate de triméthylolpropane:         10%

et sa granulométrie est de 250 à 400 μm.

Les conditions de la polymérisation sont celles de l'exemple 2.
Les conditions expérimentales de la phase d'absorption sont les suivantes:

quantité de polymère:         1 gramme
volume de solution:         4 ml
solvant:         eau-éthanol: 9/1
concentration de codéine:         20 g pour 100 ml
durée:         six heures
température:         60° C

La quantité de codéine absorbée est de 36%.
La cinétique de désorption, à pH variable, est représentée par la courbe $C_4$ de la figure 2.

### Exemple 6

La composition du polymère est la suivante:

méthacrylate de butyle:         57,5%
acide acrylique:         37,5%
triméthacrylate de triméthylolpropane:         5%

et sa granulométrie est de 200 à 400 μm.

Les conditions de la polymérisation sont celles de l'exemple 2.
Les conditions expérimentales de la phase d'absorption sont identiques à celles décrites dans l'exemple 2, à la différence près que la température est de 37° C.
La quantité de codéine absorbée est de 47%.
La cinétique de désorption, à pH variable, est représentée par la courbe $C_5$ de la figure 2.

### Exemple 7

Le polymère de même composition et même granulométrie que celui décrit dans l'exemple 6 a reçu une dose d'irradiation supplémentaire correspondant à 5 Mrad.
Les conditions expérimentales de la phase d'absorption sont identiques à celles décrites dans l'exemple 6.
La quantité de codéine absorbée atteint alors 60,5%.
La cinétique de désorption, à pH variable, est représentée par la courbe $C_6$ de la figure 2.
On a montré, par chromatographie en phase vapeur, dans le cas des exemples 3, 4, 5 et 6, qu'après désorption il n'y a aucune modification chimique de la codéine ayant été retenue sur et dans le support.
On donne à présent sept autres exemples dans lesquels la substance active est le kétoprofène.

**0 043 319**

### Exemple 8

On réalise la polymérisation en masse sous rayonnement $\gamma$ du cobalt 60 (débit de dose: 0,12 Mrad/h$^{-1}$ et dose d'irradiation: 2,6 Mrad) du mélange de monomères suivant:

| | |
|---|---|
| méthacrylate de butyle MABu: | 75 parties en poids |
| acide acrylique AA: | 15 parties en poids |
| diacrylate de tétraéthylèneglycol: | 10 parties en poids |

L'absorption du kétoprofène est réalisée sur une fraction granulométrique de 200 à 400 µm dans les conditions suivantes:

| | |
|---|---|
| solution alcoolique à 50% de kétoprofène | |
| durée d'absorption: | 24 heures |
| température d'absorption: | 20°C |
| taux d'absorption: | 27,5% de kétoprofène |

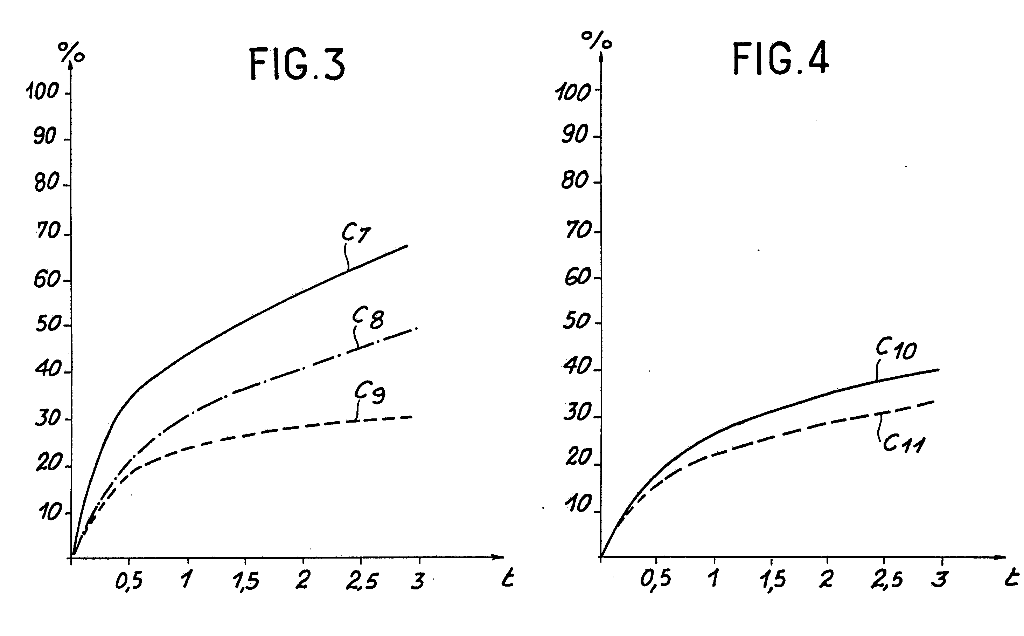

La désorption à pH constant de 7,4 est représentée par la courbe $C_7$ de la figure 3.

### Exemple 9

La polymérisation sous rayonnement $\gamma$ du cobalt 60 du mélange de monomères suivant est réalisée dans les conditions de l'exemple 8:

| | |
|---|---|
| méthacrylate de butyle MABu: | 75 parties en poids |
| acide acrylique AA: | 15 parties en poids |
| diacrylate de polyéthylèneglycol: | 10 parties en poids |

L'absorption du kétoprofène est réalisée dans les mêmes conditions que celles de l'exemple 8, sur une poudre ayant une granulométrie de 200 à 400 µm.
Le taux d'absorption est de 20% de kétoprofène.
La cinétique de désorption à pH constant de 7,4 est représentée par la courbe $C_8$ de la figure 3.

### Exemple 10

Cet exemple reprend les données de l'exemple 9 mais le diacrylate de polyéthylèneglycol est remplacé par le triacrylate de triméthylolpropane.
Le taux d'absorption est de 20% de kétoprofène.
La cinétique de désorption à pH constant de 7,4 est représentée par la courbe $C_9$ de la figure 3.

### Exemple 11

La polymérisation est effectuée sous rayonnement $\gamma$ du cobalt 60 (conditions de l'exemple 8), le mélange étant celui des monomères suivants:

| | |
|---|---|
| méthacrylate de butyle: | 79 parties en poids |
| acide acrylique: | 16 parties en poids |
| triacrylate de triméthylolpropane: | 5 parties en poids |

L'absorption du kétoprofène sur une fraction granulométrique de 200 à 400 µm est réalisée comme suit:

| | |
|---|---|
| solution alcoolique à 50% de kétoprofène | |
| durée d'absorption: | dix-sept heures |
| température d'absorption: | 37°C |
| taux d'absorption: | 32% de kétoprofène |

La cinétique de désorption à pH constant de 7,4 est représentée par la courbe $C_{10}$ de la figure 4.

8

### Exemple 12

La polymérisation et l'absorption sont effectuées dans les mêmes conditions que l'exemple 11 mais le triacrylate de triméthylolpropane est remplacé par le triméthacrylate de triméthylolpropane.

Le taux d'absorption est de 32% d kétoprofène.

La cinétique de désorption à pH constant de 7,4 est représentée par la courbe $C_{11}$ de la figure 4.

### Exemple 13

On réalise la polymérisation sous rayonnement $\gamma$ du cobalt 60 (débit de dose 0,14 Mrad/$h^{-1}$ et dose d'irradiation 3,4 Mrad) du mélange de monomères suivant:

| | |
|---|---|
| méthacrylate de butyle: | 76,5 parties en poids |
| acide acrylique: | 15,5 parties en poids |
| diacrylate de tétraéthylèneglycol: | 8 parties en poids |

L'absorption du kétoprofène est réalisée dans les conditions de l'exemple 12, sur une poudre ayant une granulométrie de 200 à 400 μm.

Le taux d'absorption est de 26% de kétoprofène.

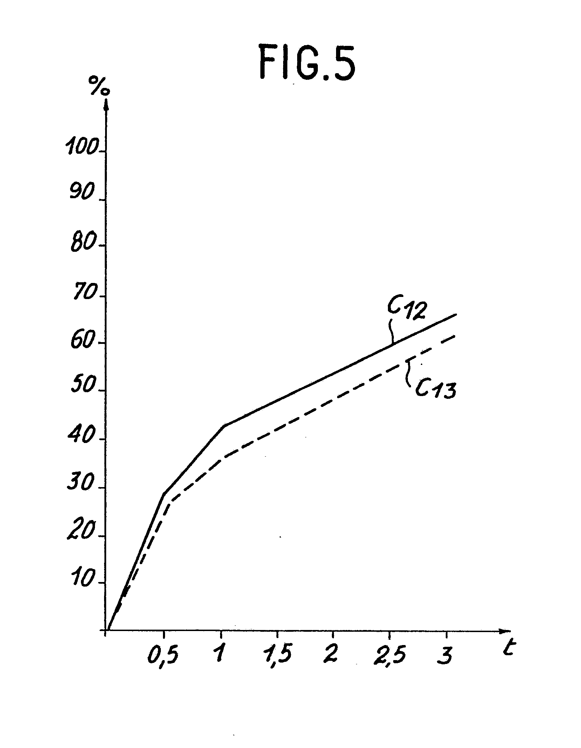

La cinétique de désorption à pH constant de 7,4 est représentée par la courbe $C_{12}$ de la figure 5.

### Exemple 14

On effectue la polymérisation sous rayonnement $\gamma$ du cobalt 60 (conditions de l'exemple 13) du mélange de monomères suivant:

| | |
|---|---|
| méthacrylate de butyle: | 75 parties en poids |
| acide acrylique: | 15 parties en poids |
| diacrylate de tétraéthylèneglycol: | 10 parties en poids |

L'absorption du kétoprofène est réalisée sous les mêmes concitions que dans l'exemple 12, la poudre de copolymère ayant une granulométrie de 200 à 400 μm.

Le taux d'absorption du kétoprofène est de 23%.

La cinétique de désorption à pH constant de 7,4 est représentée par la courbe $C_{13}$ de la figure 5.

De l'examen des courbes $C_1$ à $C_6$ (désorption à pH variable) et $C_7$ à $C_{13}$ (désorption à pH constant), on peut respectivement déduire qu'une adaptation aux besoins thérapeutiques des principes actifs pharmaceutiques donnés est possible en jouant sur la réticulation des polymères fabriqués sous rayonnements ionisants et réticulés grâce aux mécanismes d'action combinés du rayonnement et des monomères polyfonctionnels. Le débit et la dose variables du rayonnement appliqué permettent d'adapter progressivement le taux de réticulation du réseau polymère.

Les courbes des vitesses de désorption de codéine effectuées à pH variable tentent de refléter les conditions rencontrées au cours du transit intestinal. Les cinétiques de désorption, obtenues à partir des polymères fabriqués selon les modes opératoires des exemples 6, 5, 4 et 1 et testés avec des conditions d'agitation énergiques, dans un essai du genre de ceux désignés dans le métier par l'expression »diffutest«, plus particulièrement dans un diffutest EURAND, utilisé habituellement par l'industrie pharmaceutique, répondent aux normes attendues de ces médicaments de type retard pour la codéinebase.

Les courbes des vitesses de désorption du kétoprofène effectuées à pH fixe sont des tests rapides que les pharmaciens galénistes sont capables d'interpréter et de corréler au comportement du médicament »in vivo«. Ainsi, les courbes obtenues à partir des échantillons, fabriqués selon les modes opératoires des exemples 8, 13 et 14 ont été jugées satisfaisantes pour un usage thérapeutique du kétoprofène-retard

### Exemple 15

La composition du polymère est la suivante:

| | |
|---|---|
| — Méthacrylate d'heptyle: | 45% |
| — Acide acrylique: | 45% |
| — Triméthacrylate de triméthylolpropane: | 10% |

et sa granulométrie est de 250 à 400 μm.

Il a été obtenu par polymérisation en masse sous vide à un débit de dose de 0,1 Mrad · h⁻¹, la dose d'irradiation étant de 2,4 Mrad.

Les conditions expérimentales de la phase d'absorption sont les suivantes:

— Quantité de polymère:      1 gramme
— Volume de solution:        8 ml
— Solvant:                   éthanol
— Concentration de codéine:  22 g pour 100 ml
— Durée:                     18 h
— Température:               50° C

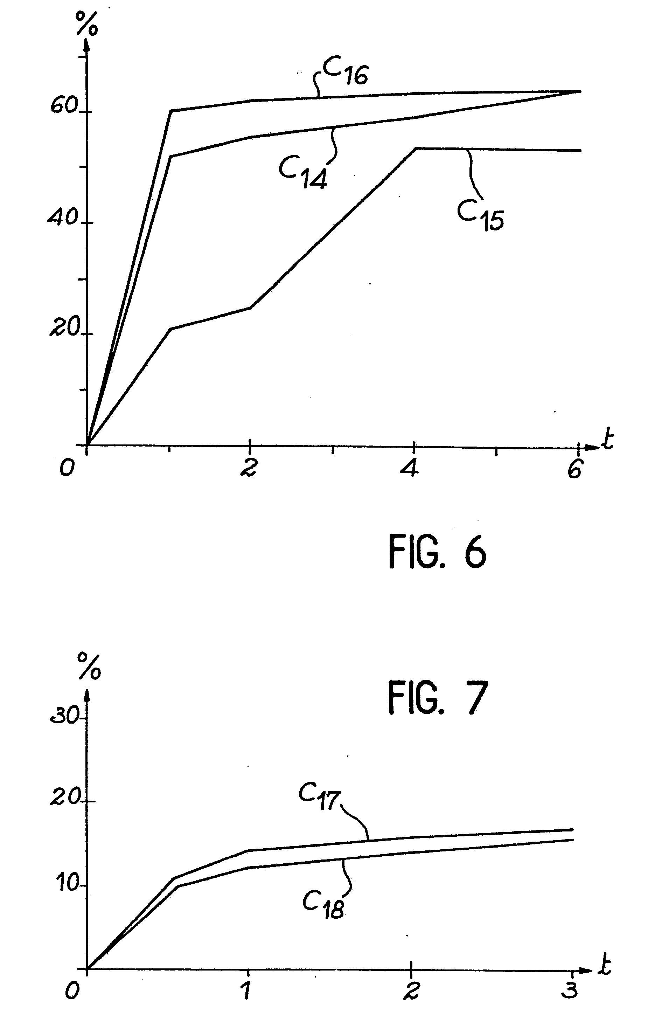

La quantité de codéine absorbée est de 52%.
La cinétique de désorption, à pH variable, est représentée par la courbe $C_{14}$ de la figure 6.

## Exemple 16

La composition du polymère est la suivante:

— Méthacrylate de butyle:                     45%
— Méthacrylate d'heptyle:                     45%
— Triméthacrylate de triméthylolproprane:     10%

et sa granulométrie est de 250 à 400 μm.

Les conditions de la polymérisation sont celles de l'exemple 15.
Les conditions expérimentales de la phase d'absorption sont identiques à celles de l'exemple 15.
La quantité de codéine absorbée est de 4,3%.
La cinétique de désorption, à pH variable est représentée par la courbe $C_{15}$ de la figure 6.

## Exemple 17

La composition du polymère est la suivante:

— Acrylamide:                        50%
— Diacrylate de polyéthylène glycol: 50%

et sa granulométrie est de 250 à 400 μm.

Il a été obtenu par polymérisation suivant des conditions identiques à celles de l'exemple 15 et réticulé sous rayonnement par une dose d'irradiation supplémentaire de 6 Mrad.
Les conditions expérimentales de la phase d'absorption sont identiques à celles de l'exemple 15.
La quantité de codéine absorbée est de 33,4%.
La cinétique de désorption, à pH variable est représentée par la courbe $C_{16}$ de la figure 6.

## Exemple 18

La polymérisation sous rayonnement $\gamma$ du Co⁶⁰ du mélange de monomères suivant est réalisée dans les conditions de l'exemple 8:

— Méthacrylate de méthyle:           47,5 parties en poids,
— Méthacrylate de butyle:            47,5 parties en poids,
— Diacrylate de polyéthylène glycol:  5 parties en poids.

L'absorption du kétoprofène est réalisée dans les mêmes conditions que celles de l'exemple 8 sur une poudre ayant une granulométrie de 200 à 400 μm.
Le taux d'absorption est de 25% de kétoprofène.
La cinétique de désorption à pH constant de 7,4 est représenté par la courbe $C_{17}$ de la figure 7.

## Exemple 19

La polymérisation sous rayonnement $\gamma$ du Co⁶⁰ du mélange de monomères suivant est réalisée dans les conditions de l'exemple 8:

- Méthacrylate de méthyle: 47,5 parties en poids,
- Méthacrylate de butyle: 47,5 parties en poids,
- Triméthacrylate de triméthylol propane: 5 parties en poids.

L'absorption de kétoprofène est réalisée dans les mêmes conditions que celles de l'exemple 8 sur une poudre ayant une granulométrie de 200 à 400 μm.

Le taux d'absorption est de 19% de kétoprofène.

La cinétique de désorption à pH constant de 7,4 est représentée par la courbe $C_{18}$ de la figure 8.

Exemple 20

Gélule à base de codéine

La quantité de médicament introduite dans la gélule est de 133 mg et comprend 40 mg de codéine-base. Le copolymère support est celui de l'exemple 2.

La granulométrie est de 250 à 400 μm.

La composition de la paroi de la gélule est la suivante:

- gélatine avec colorant contenant 12 à 15% d'eau;
- opacité obtenue par addition d'oxyde de titane;
- conservateur du type métasulfite.

Exemple 21

Suppositoire à base de codéine

133 mg du copolymère de l'exemple 2 ayant une granulométrie de 20 à 50 μm, chargé à 40 mg de codéine, sont mis en suspension homogène dans des glycérides semi-synthétiques susceptibles de fondre à 37°C. La suspension est coulée dans des moules puis refroidie.

**Revendications**

1. Support inerte en copolymère réticulé capable d'absorber puis de libérer progressivement une substance pharmaceutiquement active, caractérisé en ce qu'il est constitué par une poudre ayant une granulométrie de 20 à 700 μm, d'un copolymère réticulé comprenant:

- de 30 à 80% en poids d'acrylate et/ou de méthacrylate d'alkyle monoinsaturé;
- de 5 à 68% en poids d'acide acrylique et/ou méthacrylique; et
- de 2 à 15% en poids d'un ester acrylique et/ou méthacrylique choisi dans le groupe comprenant le diacrylate de diéthylène glycol, le diacrylate de tétraéthylène glycol (DIATEG), le diacrylate de polyéthylène glycol (DIAPEG), le triacrylate de triméthylol propane (TATMP) et le triméthacrylate de triméthylol propane (TMATMP).

2. Support selon la revendication 1, caractérisé en ce que l'acrylate ou le méthacrylate d'alkyle monoinsaturé est choisi dans le groupe comprenant les acrylates de butyle (ABu), d'hexyle et d'heptyle, et les méthacrylates de méthyle (MAM), d'éthyle, de butyle (MABu), d'hexyle et d'heptyle (MAHept).

3. Support selon l'une quelconque des revendications 1 et 2, caractérisé en ce que la poudre a une granulométrie de 100 à 400 μm.

4. Support selon l'une quelconque des revendications 1 et 2, caractérisé en ce que la poudre a une grénulométrie de 20 à 50 μm.

5. Support selon l'une quelconque des revendications 1, 3 et 4, caractérisé en ce que le copolymère est un copolymère de méthacrylate de butyle, d'acide acrylique et de triacrylate de triméthylol propane.

6. Support selon l'une quelconque des revendications 1, 3 et 4, caractérisé en ce que le copolymère est un copolymère de méthacrylate de butyle, d'acide acrylique et de diacrylate de tétraéthylène glycol.

7. Support selon l'une quelconque des revendications 1, 3 et 4, caractérisé en ce que le copolymère est un copolymère de méthacrylate d'heptyle, d'acide acrylique et de triméthacrylate de triméthylol propane.

8. Support selon l'une quelconque des revendications 1, 3 et 4, caractérisé en ce que le copolymère est un copolymère de méthacrylate de butyle, d'acide acrylique et de triméthacrylate de triméthylol propane.

9. Composition pharmaceutique comprenant une substance pharmaceutiquement active et un support inerte, caractérisée en ce que la substance pharmaceutiquement active est la codéine, et en ce que le support inerte est une poudre ayant une granulométrie de 20 à 700 µm, d'un copolymère de 30 à 80% en poids de méthacrylate d'heptyle, de 5 à 68% en poids d'acide acrylique et de 2 à 15% en poids de triméthacrylate de triméthylol propane.

10. Composition pharmaceutique comprenant une substance pharmaceutiquement active et un support inerte, caractérisée en ce que la substance pharmaceutiquement active est la codéine, et en ce que le support inerte est une poudre ayant une granulométrie de 20 à 700 µm d'un copolymère de 30 à 80% en poids de méthacrylate de butyle, de 5 à 68% en poids d'acide acrylique et de 2 à 15% en poids de triméthacrylate de triméthylol propane.

11. Composition pharmaceutique comprenant une substance pharmaceutiquement active et un support inerte, caractérisée en ce que la substance pharmaceutiquement active est l'acide 2-(3-benzoylphényl)-propionique et en ce que la substance inerte est une poudre ayant une granulométrie de 20 à 700 µm, d'un copolymère de 30 à 80% en poids de méthacrylate de butyle, de 5 à 68% en poids d'acide acrylique et de 2 à 15% en poids de triacrylate de triméthylol propane.

12. Composition pharmaceutique comprenant une substance pharmaceutiquement active et un support inerte, caractérisée en ce que la substance pharmaceutiquement active est l'acide 2-(3-benzoylphényl)-propionique, et en ce que le support inerte est une poudre ayant une granulométrie de 20 à 700 µm, d'un copolymère de 30 à 80% en poids de méthacrylate de butyle, de 5 à 68% en poids d'acide acrylique et de 2 à 15% en poids de diacrylate de tétraéthylène glycol.

13. Composition pharmaceutique comprenannt une substance pharmaceutiquement active et un support inerte, caractérisée en ce que la substance pharmaceutiquement active est l'acide 2-(3-benzoylphényl)-propionique, et en ce que le support inerte est une poudre ayant une granulométrie de 20 à 700 µm, d'un copolymère de 30 à 80% en poids de méthacrylate de butyle, 5 à 68% en poids d'acide acrylique et de 2 à 15% en poids de triméthacrylate de triméthylol propane.

14. Procédé de préparation d'un support inerte selon l'une quelconque des revendications 1 à 8, caractèrisé en ce que l'on soumet à une irradiation ionisante un mélange de monomères comprenant:

— de 30 à 80% en poids d'un acrylate et/ou d'un méthacrylate d'alkyle monoinsaturé,
— 5 à 68% d'acide acrylique et/ou méthacrylique, et
— 2 à 15% en poids d'un ester acrylique et/ou méthacrylique choisi dans le groupe comprenant le diacrylate de diéthylène glycol, le diacrylate de tétraéthylène glycol, le diacrylate de polyéthylène glycol, le triacrylate de triméthylol propane et le triméthacrylate de triméthylol propane de façon à copolymériser et réticuler lesdits monomères.

15. Procédé selon la revendication 14, caractérisé en ce que l'on réalise la polymérisation et la réticulation en émulsion.

16. Procédé selon l'une quelconque des revendications 14 et 15, caractérisé en ce que l'on adapte le taux de réticulation du copolymère obtenu en le soumettant à une irradiation complémentaire au moyen de rayonnements ionisants.

17. Procédé selon l'une quelconque des revendications 14 à 16, caractérisé en ce que la dose d'irradiation intégrée utilisée pour réaliser la polymérisation et la réticulation est d'environ 9000 à 80 000 Gy.

**Patentansprüche**

1. Inerter Träger aus einem vernetzten Copolymeren, der eine pharmazeutisch aktive Substanz absorbieren und dann allmählich wieder freisetzen kann, dadurch gekennzeichnet, daß er besteht aus einem Pulver eines vernetzten Copolymeren mit einer Korngrößenverteilung von 20 bis 700 µm, das enthält:

— 30 bis 80 Gew.-% einfach ungesättigtes Alkylacrylat und/oder -methacrylat;
— 5 bis 68 Gew.-% Acrylsäure und/oder Methacrylsäure; und
— 2 bis 15 Gew.-% eines Acrylsäure- und/oder Methacrylsäureesters, ausgewählt aus der Gruppe, die umfaßt Diäthylenglykoldiacrylat, Tetraäthylenglykoldiacrylat (DIATEG), Polyäthylenglykoldiacrylat (DIAPEG), Trimethylolpropantriacrylat (TATMP) und Trimethylolpropantrimethacrylat (TMATMP).

2. Träger nach Anspruch 1, dadurch gekennzeichnet, daß das einfach ungesättigte Alkylacrylat oder Alkylmethacrylat ausgewählt wird aus der Gruppe, die umfaßt die Butyl-(ABu), Hexyl- und Heptylacrylate und die Methyl-(MAM), Äthyl-, Butyl-(MABu), Hexyl- und Heptylmethacrylate (MAHept).

3. Träger nach einem der Ansprüche 1 und 2, dadurch gekennzeichnet, daß das Pulver eine Korngrößenverteilung von 100 bis 400 μm hat.

4. Träger nach einem der Ansprüche 1 und 2, dadurch gekennzeichnet, daß das Pulver eine Korngrößenverteilung von 20 bis 50 μm hat.

5. Träger nach einem der Ansprüche 1, 3 und 4, dadurch gekennzeichnet, daß das Copolymere ein Colpolymeres von Butylmethacrylat, Acrylsäure und Trimethylolpropantriacrylat ist.

6. Träger nach einem der Ansprüche 1, 3 und 4, dadurch gekennzeichnet, daß das Copolymere ein Copolymeres von Butylmethacrylat, Acrylsäure und Tetraäthylenglykoldiacrylat ist.

7. Träger nach einem der Ansprüche 1, 3 und 4, dadurch gekennzeichnet, daß das Copolymere ein Copolymeres von Heptylmethacrylat, Acrylsäure und Trimethylolpropantrimethacrylat ist.

8. Träger nach einem der Ansprüche 1, 3 und 4, dadurch gekennzeichnet, daß das Copolymere ein Copolymeres von Butylmethacrylat, Acrylsäure und Trimethylolpropantrimethacrylat ist.

9. Pharmazeutische Zusammensetzung, die eine pharmazeutisch aktive Substanz und einen inerten Träger enthält, dadurch gekennzeichnet, daß die pharmazeutisch aktive Substanz Codein ist und daß der inerte Träger ein Pulver aus einem Copolymeren aus 30 bis 80 Gew.-% Hexylmethacrylat, 5 bis 68 Gew.-% Acrylsäure und 2 bis 15 Gew.-% Trimethylolpropantrimethacrylat mit einer Korngrößenverteilung von 20 bis 700 μm ist.

10. Pharmazeutische Zusammensetzung, die eine pharmazeutisch aktive Substanz und einen inerten Träger enthält, dadurch gekennzeichnet, daß die pharmazeutisch aktive Substanz Codein ist und daß der inerte Träger ein Pulver eines Copolymeren aus 30 bis 80 Gew.-% Butylmethacrylat, 5 bis 68 Gew.-% Acrylsäure und 2 bis 15 Gew.-% Trimethylolpropantrimethacrylat mit einer Korngrößenverteilung von 20 bis 700 μm ist.

11. Pharmazeutische Zusammensetzung, die eine pharmazeutisch aktive Substanz und einen inerten Träger enthält, dadurch gekennzeichnet, daß die pharmazeutisch aktive Substanz die 2-(3-Benzoylphenyl)-propionsäure ist und daß die inerte Substanz ein Pulver eines Copolymeren aus 30 bis 80 Gew.-% Butylmethacrylat, 5 bis 68 Gew.-% Acrylsäure und 2 bis 15 Gew.-% Trimethylolpropantriacrylat mit einer Korngrößenverteilung von 20 bis 700 μm ist.

12. Pharmazeutische Zusammensetzung, die eine pharmazeutisch aktive Substanz und einen inerten Träger enthält, dadurch gekennzeichnet, daß die pharmazeutisch aktive Substanz die 2-(3-Benzoylphenyl)-propionsäure ist und daß der inerte Träger ein Pulver eines Copolymeren aus 30 bis 80 Gew.-% Butylmethacrylat, 5 bis 68 Gew.-% Acrylsäure und 2 bis 15 Gew.-% Tetraäthylenglykoldiacrylat mit einer Korngrößenverteilung von 20 bis 700 μm ist.

13. Pharmazeutische Zusammensetzung, die eine pharmazeutisch aktive Substanz und einen inerten Träger enthält, dadurch gekennzeichnet, daß die pharmazeutisch aktive Substanz die 2-(3-Benzoylphenyl)-propionsäure ist und daß der inerte Träger ein Pulver eines Copolymeren aus 30 bis 80 Gew.-% Butylmethacryiat, 5 bis 68 Gew.-% Acrylsäure und 2 bis 15 Gew.-% Trimethylolpropantrimethacrylat mit einer Korngrößenverteilung von 20 bis 700 μm ist.

14. Verfahren zur Herstellung eines inerten Trägers nach einem der Ansprüche 1 bis 8, dadurch gekennzeichnet, daß man ein Monomerengemisch, das enthält:

—  30 bis 80 Gew.-% eines einfach ungesättigten Alkylacrylats und/oder -methacrylats,
—  5 bis 68 Gew.-% Acrylsäure und/oder Methacrylsäure und
—  2 bis 15 Gew.-% eines Acrylsäureesters und/oder Methacrylsäureesters, ausgewählt aus der Gruppe, die umfaßt das Diäthylenglykoldiacrylat, das Tetraäthylenglykoldiacrylat, das Polyäthylenglykoldiacrylat, das Trimethylolpropantriacrylat und das Trimethylolpropantrimethacrylat,

einer ionisierenden Bestrahlung aussetzt, um die genannten Monomeren zu copolymerisieren und zu vernetzen.

15. Verfahren nach Anspruch 14, dadurch gekennzeichnet, daß man die Polymerisation und die Vernetzung in Emulsion durchführt.

16. Verfahren nach einem der Ansprüche 14 und 15, dadurch gekennzeichnet, daß man den Vernetzungsgrad des erhaltenen Copolymeren anpaßt, indem man es einer komplementären Bestrahlung mit ionisierenden Strahlen aussetzt.

17. Verfahren nach einem der Ansprüche 14 bis 16, dadurch gekennzeichnet, daß die zur Durchführung der Polymerisation und der Vernetzung angewendete Gesamtbestrahlungsdosis etwa 9000 bis 80 000 Gy beträgt.

## Claims

1. Inert carrier comprising a crosslinked polymer capable of absorbing and subsequently progressively releasing a pharmaceutically active substance, characterized in that it comprises a powder, having a particle size from 20 to 700 μm, of a crosslinked polymer comprising:

—  from 30 to 80% by weight of a monounsaturated alkyl acrylate and/or methacrylate;

—  from 5 to 68% by weight of acrylic and/or methacrylic acid; and

—  from 2 to 15% by weight of an acrylic and/or methacrylic ester selected from the group comprising diethylene glycol diacrylate, tetraethylene glycol diacrylate (DIATEG), polyethylene glycol diacrylate (DIAPEG), trimethylolpropane triacrylate (TATMP) and trimethylolpropane trimethacrylate (TMATMP).

2. Carrier according to Claim 1 characterized in that the monounsaturated alkyl acrylate or methacrylate is selected from the group comprising butyl (ABu), hexyl and heptyl acrylates, and methyl (MAM), ethyl, butyl (MABu), hexyl and heptyl (MAHept) methacrylates.

3. Carrier according to either of Claims 1 and 2 characterized in that the powder has a particle size from 100 to 400 μm.

4. Carrier according to either of Claims 1 and 2 characterized in that the powder has a particle size from 20 to 50 μm.

5. Carrier according to any one of Claims 1, 3 and 4 characterized in that the copolymer is a copolymer of butyl methacrylate, acrylic acid and trimethylolpropane triacrylate.

6. Carrier according to any one of Claims 1, 3 and 4 characterized in that the copolymer is a copolymer of butyl methacrylate, acrylic acid and tetraethylene glycol diacrylate.

7. Carrier according to any one of Claims 1, 3 and 4 characterized in that the copolymer is a copolymer of heptyl methacrylate, acrylic acid and trimethylolpropane trimethacrylate.

8. Carrier according to any one of Claims 1, 3 and 4 characterized in that the copolymer is a copolymer of butyl methacrylate, acrylic acid, and trimethylolpropane trimethacrylate.

9. Pharmaceutical composition comprising a pharmaceutically-active substance and an inert carrier, characterized in that the pharmaceuticallyactive substance is codeine and in that the inert carrier is a powder having a particle size of 20 to 700 μm, of a copolymer of 30 to 80% by weight of heptyl methacrylate, 5 to 68% by weight of acrylic acid and 2 to 15% by weight of trimethylolpropane trimethacrylate.

10. Pharmaceutical composition comprising a pharmaceutically-active substance and an inert carrier, characterized in that the pharmaceuticallyactive substance is codeine and in that the inert carrier is a powder having a particle size of 20 to 700 μm, of a copolymer of 30 to 80% by weight of butyl methacrylate, 5 to 68% by weight of acrylic acid and 2 to 15% by weight of trimethylolpropane trimethacrylate.

11. Pharmaceutical composition comprising a pharmaceutically-active substance and an inert carrier, characterized in that the pharmaceuticallyactive substance is 2-(3-benzoylphenyl) propionic acid and in that the inert carrier is a powder having a particle size of 20 to 700 μm, of a copolymer of 30 to 80% by weight of butyl methacrylate, 5 to 68% by weight of acrylic acid and 2 to 15% by weight of trimethylolpropane triacrylate.

12. Pharmaceutical composition comprising a substance and an inert carrier, characterized in that the pharmaceutically-active substance is 2-(3-benzoylphenyl) propionic acid and in that the inert carrier is a powder having a particle size of 20 to 700 μm, of a copolymer of 30 to 80% by weight of butyl methacrylate, 5 to 68% by weight of acrylic acid and 2 to 15% by weight of tetraethylene glycol diacrylate.

13. Pharmaceutical composition comprising a pharmaceutically-active substance and an inert carrier, characterized in that the pharmaceutically-active substance is 2-(3-benzoylphenyl) propionic acid and in that the inert carrier is a powder having a particle size of 20 to 700 μm, of a copolymer of 30 to 80% by weight of butyl methacrylate, 5 to 68% by weight of acrylic acid and 2 to 15% by weight of trimethylolpropane trimethacrylate.

14. Process for the preparation of an inert carrier according to any one of Claims 1 to 8 characterized in that a monomer mixture comprising:

—  from 30 to 80% by weight of a monounsaturated alkyl acrylate or methacrylate;

—  from 5 to 68% by weight of acrylic and/or methacrylic acid; and

—  from 2 to 15% by weight of an acrylic and/or methacrylic ester selected from the group comprising diethylene glycol diacrylate, tetraethylene glycol diacrylate, polyethylene glycol diacrylate, trimethylolpropane triacrylate and trimethylol propane trimethacrylate,

is subjected to ionizing radiation whereby to copolymerize and crosslink said monomers.

15. Process according to Claim 14 characterized in that the polymerization and crosslinking are conducted in emulsion.

16. Process according to either of Claims 14 and 15 characterized in that the degree of crosslinking of the resulting copolymer is adjusted by submitting it to supplementary irradiation with ionizing radiation.

17. Process according to any one of Claims 14 to 16 characterized in that the total radiation dose employed for polymerization and crosslinking is about 9000 to 80 000 Gy.

FIG.1

FIG.2

FIG.3

FIG.4

# FIG.5

FIG. 6

FIG. 7